# EUROPÄISCHE PATENTANMELDUNG

(11) **EP 2 631 267 A1**
(43) Veröffentlichungstag der Anmeldung: **28.08.2013**
(21) Anmeldenummer: 12156808.3
(22) Anmeldetag: 24.02.2012
(51) Int. Cl.: C08K 5/11

(54) **Bernsteinsäurealkylester-Mischungen als Weichmacher**

(71) Anmelder: Lanxess Deutschland GmbH, 51369 Leverkusen (DE)
(72) Erfinder: FACKLAM, Thomas, 51375 Leverkusen (DE)

(57) **Zusammenfassung**

Die vorliegende Erfindung betrifft neue Bernsteinsäurealkylester Mischungen und deren Verwendung als Weichmacher für Kunststoffe. Die erfindungsgemäßen Mischungen zeichnen sich durch gute weichmachende Wirksamkeit aus und führen bei den daraus hergestellten Produkten zu verbesserten Gebrauchseigenschaften.

## Beschreibung

Die vorliegende Erfindung betrifft neue Bernsteinsäurealkylester Mischungen und deren Verwendung als Weichmacher für Kunststoffe.

Zur Verarbeitung von Kunststoffen wie beispielsweise Polyvinylchlorid (PVC) werden seit Jahrzehnten Weichmacher eingesetzt. Weichmacher sind in der Polymerverarbeitung eingesetzte Additive, die die Verarbeitbarkeit, die Flexibilität und die Dehnbarkeit verbessern. Da die Weichmacher nicht fest mit dem Polymeren verbunden sind, können sie migrieren oder sich verflüchtigen. Die zur Erzeugung von Weich-PVC eingesetzten Weichmacher sind überwiegend Phthalsäureester wie die Allzweckprodukte Di-2-ethylhexylphthalat (DEHP), Düsononylphthalat (DINP) und Diisodecylphthalat (DIDP). Zur Verbesserung der Verarbeitungsprozesse in Hinblick auf Geschwindigkeit oder Energieeinsparung können schnell gelierende Weichmacher wie beispielsweise die kurzkettigen Phthalate Dibutylphthalat (DBP), Diisobutylphthalat (DiBP) und Benzylbutylphthalat (BBP) zugesetzt werden.

Die Verwendung von Phthalaten wird aufgrund gesetzlicher Rahmenbedingungen immer weiter eingeschränkt. Ein Beispiel ist das Verbot und die Beschränkung des Einsatzes einiger Phthalate zur der Herstellung von Spielzeug und Babyartikeln (Richtlinie 2005/84/EG des Europäischen Parlaments und des Rates vom 14. Dezember 2005). Auch wurden von der Europäischen Chemikalienagentur (ECHA) mehrere Phthalate in die Liste der Kandidaten der besonders Besorgnis erregenden Stoffe (SVHC) aufgenommen.

Hinzu kommt, dass die Phthalate-Weichmacher auf petrochemisch erzeugten Rohstoffen basieren. Bei deren Herstellung werden Treibhausgase frei gesetzt was in vieler Hinsicht als problematisch anzusehen ist.

Deshalb besteht ein Bedarf an phthalatfreien Weichmachern für Kunststoffe. Weichmacher auf Basis nachwachsender Rohstoffe stehen dabei wegen der Diskussion zu Klima, Nachhaltigkeit und begrenzter Verfügbarkeit fossiler Rohstoffe im Fokus des Interesses. Weichmacher auf Basis von Bernsteinsäure könnten diese Anforderungen erfüllen.

Die Verwendung von Bernsteinsäureestern als Weichmacher für Kunststoffe ist bereits seit längerer Zeit bekannt. So beschreibt z.B. die DE-A 1962500 die Verwendung von Dialkylsuccinaten, insbesondere von Bernsteinsäuredicaprylester, als Weichmacher für fleckenbeständige Folien aus PVC.

Da neuere Entwicklungen auf dem Gebiet der "grünen" Technologien einen kostengünstigen und effektiven Zugang zu bio-basierter Bernsteinsäure geschaffen haben, ist der Einsatz von bio-basierter Bernsteinsäure zur Herstellung von Weichmachern in technischem Maßstab möglich geworden. In diesem Zusammenhang wurde die Bernsteinsäure als einer von 12 zuckerbasierten Synthesebausteinen von höchstem Wert identifiziert (vgl. z.B. T. Werpy and G. Petersen et al. in Top Value Added Chemicals From Biomass, Volume I: Results of Screening for Potential Candidates from Sugars and Synthesis Gas, Seite 1)

In Bezug auf Nachhaltigkeit und Verwendung von Chemikalien aus nachwachsenden Rohstoffen sind insbesondere die Ester der Bernsteinsäure mit Fettalkoholen wie, nicht einschränkend, 1-Octanol, 1-Decanol und 1-Dodecanol, von hohem Interesse da auch die genannten Alkohole auf dem bio-Weg, zugänglich sind, z.B. durch Hydrierung von Fettsäuren aus pflanzlichen Ölen.

Die Verwendung von Estern der Bernsteinsäure als Weichmacher für PVC wird von LeCaptain et al. in Polym. Bull. (2010) 65:589-598 im Aufsatz "Poly(vinyl chloride) plasticized with succinate esters: synthesis and characterization" dargestellt. Dort werden Di-octyl succinat (DOS), Di-hexyl succinate (DHS), Di-butyl succinate (DBS) und Di-ethyl succinate (DES) beschrieben. Durch Infrarot (IR)-, dynamische Differenzkalorimetrie (DSC)-, und dynamisch-mechanische Analyse (DMA) wird die Verträglichkeit der Ester in PVC und deren Potential als Ersatz von Phthalaten qualitativ festgestellt. Für den Verarbeiter wichtige anwendungstechnische Untersuchungen und Aussagen wie Migration und Flüchtigkeit aus dem weichgemachten Polymeren oder dessen Langzeitgebrauchseigenschaften werden nicht gemacht.

Untersuchungen ergeben, dass Di-n-Octyl-succinat (CAS Nr 14491-66-8) eine gute Weichmachereffektivität in Bezug auf Erniedrigung der Härte besitzt. Allerdings ist die Flüchtigkeit der Chemikalie höher als die des entsprechenden Adipinsäureesters was mit dem geringeren Molekulargewicht des Succinates erklärt werden kann. Das Di-n-Decyl-succinat (CAS Nr. 10595-82-1 ) hat keine gute weichmachende Wirkung; möglicherweise ist dies die Ursache des Schmelzpunktes von > 20°C und der Kristallisation des festen Weichmachers im Endprodukt. Das Di-n-Dodecylsuccinat (CAS Nr 5980-15-5) ist bei Raumtemperatur ein Feststoff mit mangelhafter Verarbeitbarkeit.

Die genannten, aus dem Stand der Technik bekannten Bernsteinsäureester entsprechen nicht in allen Punkten dem gewünschten Anforderungsprofil eines guten Weichmachers, insbesondere in Bezug auf Weichmachereffektivität und lange Nutzungsdauer der Endprodukte, und sind in dieser Hinsicht noch verbesserungsbedürftig.

Ausgehend vom bekannten Stand der Technik bestand die Aufgabe der vorliegenden Erfindung darin, neue Weichmacher für Kunststoffe auf Basis von Bernsteinsäurealkylestern bereit zu stellen, die über verbesserte Eigenschaften verfügen, insbesondere in Bezug auf gute weichmachende Wirkung in Verbindung mit langer Nutzungsdauer des Endartikels.

Es wurde nun gefunden, dass Mischungen von wenigstens zwei Bernsteinsäurealkylestern, basierend auf zwei unterschiedlichen, monofunktionellen Alkoholen, als Weichmacher für Kunststoffe eingesetzt werden können. Die erfindungsgemäßen Mischungen zeichnen sich durch gute weichmachende Wirksamkeit aus und führen überraschenderweise bei den daraus hergestellten Produkten zu verbesserten Gebrauchseigenschaften.

Gegenstand der vorliegenden Erfindung sind Mischungen von Bernsteinsäurealkylestern dadurch gekennzeichnet, dass sie mindestens zwei Verbindungen ausgewählt aus den Formeln

R¹-OC(O)-CH₂-CH₂-C(O)O-R¹ (I)

R¹-OC(O)-CH₂-CH₂-C(O)O-R² (II)

und

R²-OC(O)-CH₂-CH₂-C(O)O-R² (III)

enthalten,
worin
R¹ und R² jeweils für einen geradkettigen oder verzweigten Alkylrest stehen,
mit der Maßgabe, dass R¹ nicht gleich R² ist.
Bevorzugt sind Mischungen von Bernsteinsäurealkylestern wobei in den Formeln (I), (II) und (III)
R¹ und R² jeweils für einen geradkettigen oder verzweigten Alkylrest mit 1 bis 12 Kohlenstoffatomen stehen.
Besonders bevorzugt sind Mischungen von Bernsteinsäurealkylestern wobei in den Formeln (I), (II) und (III)
R¹ und R² jeweils für einen geradkettigen oder verzweigten Alkylrest mit 8 bis 12 Kohlenstoffatomen stehen.
Ganz besonders bevorzugt sind Mischungen von Bernsteinsäurealkylestern wobei in den Formeln (I), (II) und (III)

R¹ und R² jeweils für einen geradkettigen oder verzweigten Alkylrest mit 8, 9 oder 10 Kohlenstoffatomen stehen.

Als geradkettige oder verzweigte Alkylreste kommen beispielsweise in Frage: Methyl-; Ethyl-; Propyl- wie n-Propyl-, iso-Propyl-; Butyl- wie n-Butyl-, sek.-Butyl-, Isobutyl-; Amyl-; Hexyl-, wie n-Hexyl-, 1,4-Dimethylbutyl-; n-Heptyl-; Octyl- wie Isooctyl-, n-Octyl-, 2-Ethylhexyl-; Nonyl- wie n-Nonyl- und Isononyl-; Decyl-. wie n-Decyl-, Isodecyl-; und Dodecyl- wie n-Dodecyl- und Isododecyl und deren gesamte verschiedene isomere Formen.

Insbesondere bevorzugt sind Mischungen von Bernsteinsäurealkylestern worin in den Formeln (I), (II) und (III) R¹ für n-Octyl und R² für n-Decyl steht.

Weiterhin besonders bevorzugt sind a) Mischungen von Bernsteinsäurealkylestern die dadurch gekennzeichnet sind, dass sie die Verbindungen der Formeln (I), (II) und (III) enthalten. In den Mischungen a) haben die Reste R¹ und R² jeweils die oben angegebenen allgemeinen und bevorzugten Bedeutungen, wobei die Reste R¹ und R² nicht gleich sind. Von den Mischungen a) sind wiederum solche ganz besonders bevorzugt, worin in den Formeln (I), (II) und (III) R¹ für n-Octyl und R² für n-Decyl steht.

In den Mischungen a) beträgt die Menge an Verbindung der Formel (I) im allgemeinen 10 bis 50 Gew.-% bevorzugt 15 bis 35 Gew.-% und ganz besonders bevorzugt 20 bis 30 Gew.-%, die Menge an Verbindung der Formel (II) im allgemeinen 25 bis 75 Gew.-%, bevorzugt 35 bis 65 Gew.-% und ganz besonders bevorzugt 40 bis 60 Gew.-%, und die Menge an Verbindung der Formel (III) im allgemeinen 10 bis 50 Gew.-% bevorzugt 15 bis 35 Gew.-% und ganz besonders bevorzugt 20 bis 30 Gew.-% jeweils bezogen auf 100 Prozent der Mischung.

Ebenfalls besonders bevorzugt sind b) Mischungen von Bernsteinsäurealkylestern die dadurch gekennzeichnet sind, dass sie die Verbindungen der Formeln (I) und (III) enthalten. In den Mischungen b) haben die Reste R¹ und R² jeweils die oben angegebenen allgemeinen und bevorzugten Bedeutungen, wobei die Reste R¹ und R² nicht gleich sind. Von den Mischungen b) sind wiederum solche ganz besonders bevorzugt, worin in den Formeln (I) und (III) R¹ für n-Octyl und R² für n-Decyl steht.

In den Mischungen b) beträgt die Menge an Verbindung der Formel (I) im allgemeinen 15 bis 95 Gew.-% bevorzugt 25 bis 75 Gew.-% und ganz besonders bevorzugt 40 bis 60 Gew.-%, die Menge an Verbindung der Formel (III) im allgemeinen 85 bis 5 Gew.-%, bevorzugt 75 bis 25 Gew.-% und ganz besonders bevorzugt 60 bis 40 Gew.-%, jeweils bezogen auf 100 Prozent der Mischung.

Die Verbindungen der Formel (II) sind neu und ebenfalls Gegenstand der vorliegenden Erfindung. Bevorzugt sind solche Verbindungen der Formel (II) worin R¹ für n-Octyl und R² für n-Decyl steht. Die Verbindungen (II) eignen sich hervorragend als Weichmacher für Kunststoffe.

Die erfindungsgemäßen Mischungen können nach unterschiedlichen Verfahren hergestellt werden. So ist es beispielweise möglich zwei unterschiedliche monofunktionelle Alkohole der Formeln R¹-OH (IV) und R²-OH (V), worin R¹ und R² die oben, für die Formeln (I) bis (III) angegebenen, allgemeinen und bevorzugten Bedeutungen haben, mit Bernsteinsäure, in an sich bekannter Weise, unter Abspaltung von Wasser umzusetzen. Das Verfahren kann in einem oder in zwei Schritten durchgeführt werden. Bei der Durchführung in einem Schritt werden alle Reaktionsteilnehmer im Wesentlichen gleichzeitig miteinander in Kontakt gebracht und umgesetzt. Bei der Umsetzung in zwei Schritten werden in einem ersten Schritt die Bernsteinsäure oder ein Derivat davon mit einem Alkohol umgesetzt und die dabei erhaltene Reaktionsmischung mit dem zweiten Alkohol umgesetzt. Die Reaktionsmischung kann mit einem Lösemittel, das auch als Schleppmittel zur Ausschleusung von Reaktionswasser dienen kann, verdünnt werden. Die zur Bildung des Esters eingesetzten monofunktionellen Alkohole können gleichzeitig als Schleppmittel und im Überschuss eingesetzt werden. Die Veresterung der Bernsteinsäure kann mit oder ohne typische, dem Fachmann geläufige Katalysatoren durchgeführt werden.

Auch ist es möglich, durch Reaktion der Alkohole der Formeln (IV) und (V) mit Bernsteinsäurehalogeniden unter Abspaltung von Halogenwasserstoffsäure die erfindungsgemäßen Mischungen herzustellen. Dabei können die Alkohole der Formeln (IV) und (V) entweder gleichzeitig oder nacheinander zur Reaktion gebracht werden. Weiterhin ist es möglich durch Umesterung eines Bernsteinsäureesters mit kurzkettigen Alkoholen, beispielsweise Bernsteinsäuredimethylester, mit den Alkoholen der Formeln (IV) und (V) unter Abspaltung von beispielsweise Methanol, oder durch Hydrierung des entsprechenden Estergemisches der Fumar- oder Maleinsäure oder auf einem anderen Wege, wie beispielsweise durch Mischung der Einzelkomponenten (I), (II) und (III) in unterschiedlichen Mengen, die erfindungsgemäßen Mischungen herzustellen.

Den oben beschriebenen Umsetzungen können sich dem Fachmann geläufige Reinigungsoperationen, wie zum Beispiel Extraktion, insbesondere wässrige Wäsche, Destillation, Wasserdampfdestillation, Adsorption und / oder Filtration anschließen.

Weiterer Gegenstand der vorliegenden Erfindung ist ein Verfahren zur Herstellung einer erfindungsgemäßen Estermischung enthaltend die Verbindungen der Formeln

R¹-OC(O)-CH₂-CH₂-C(O)O-R¹ (I),

R¹-OC(O)-CH₂-CH₂-C(O)O-R² (II)

und

R²-OC(O)-CH₂-CH₂-C(O)O-R² (III),

worin

R¹ und R² jeweils für einen geradkettigen oder verzweigten Alkylrest stehen,
mit der Maßgabe, dass R¹ nicht gleich R² ist,
das dadurch gekennzeichnet ist, dass man in einem Verfahrensschritt oder in zwei aufeinander folgenden Verfahrensschritten zwei unterschiedliche monofunktionelle Alkohole der Formeln R¹-OH (IV) und R²-OH (V), worin R¹ und R² die oben, für die Formeln (I) bis (III) angegebenen, allgemeinen und bevorzugten Bedeutungen haben, bei einer Temperatur von 50 bis 250°C und gegebenenfalls bei einem Druck von 2 mbar bis 4 bar und gegebenenfalls in Gegenwart eines Katalysators mit Bernsteinsäure umsetzt und das gebildete Reaktionswasser durch geeignete Maßnahmen, beispielsweise Destillation, aus der Mischung entfernt

Als Alkohole der Formeln (IV) und (V) kommen beispielsweise in Frage: Methyl-; Ethyl-; Propylwie n-Propyl-, iso-Propyl-; Butyl- wie n-Butyl-, sek.-Butyl-, Isobutyl-; Amyl-; Hexyl-, wie n-Hexyl-, 1,4-Dimethylbutyl-; n-Heptyl-; Octyl- wie Isooctyl-, n-Octyl-, 2-Ethylhexyl-; Nonyl- wie n-Nonyl-und Isononyl-; Decyl-. wie n-Decyl-, Isodecyl-; und Dodecyl- wie n-Dodecyl- und Isododecylalkohol und deren gesamte verschiedene isomere Formen.

Als Katalysatoren eignen sich prinzipiell Verbindungen der Formel MXn worin M für ein Metallkation steht, ausgewählt aus der Gruppe der Metalle Titan, Zirkonium, Vanadium, Aluminium, Eisen, Zinn, und X für ein Anion ausgewählt aus der Gruppe, -CO₃²⁻, Cl⁻, Br⁻, I⁻; -OR⁻ mit R ausgewählt aus Methyl, Ethyl, n-Propyl, i-Propyl, n-Butyl, i-Butyl, t-Butyl; Carboxylate insbesondere Hexanoat, Heptanoat, Octanoat, 2-Ethylhexanoat, Stearat, Palmitat, Oxalat; und worin n für die Oxidationszahl des Metalls, bevorzugt für 2, 3 oder 4 steht. Alternativ können auch starke Brönstedsäuren wie Schwefelsäure, saure Sulfate, wie z.B. Methylsulfat, Ethylsulfat, Propylsulfat, Butylsulfat, Hexylsulfat aber auch KHSO₄ bzw. NaHSO₄, aromatische Sulfonsäuren insbesondere para-Toluolsulfonsäure, Benzolsulfonsäure erfolgreich eingesetzt werden.

Ebenfalls Gegenstand der vorliegenden Erfindung ist ein Verfahren zur Herstellung einer erfindungsgemäßen Estermischung enthaltend die Verbindungen der Formeln

R¹-OC(O)-CH₂-CH₂-C(O)O-R¹ (I)

und

R²-OC(O)-CH₂-CH₂-C(O)O-R² (III),

worin

R¹ und R² jeweils für einen geradkettigen oder verzweigten Alkylrest stehen,
mit der Maßgabe, dass R¹ nicht gleich R² ist, das dadurch gekennzeichnet ist, dass man die einzelnen Verbindungen der Formeln (I) und (II) miteinander vermischt.

Ebenfalls Gegenstand der vorliegenden Erfindung ist ein Verfahren zur Herstellung von Verbindungen der Formel

R¹-OC(O)-CH₂-CH₂-C(O)O-R² (II)

worin

R¹ und R² jeweils für einen geradkettigen oder verzweigten Alkylrest stehen,
mit der Maßgabe, dass R¹ nicht gleich R² ist, das dadurch gekennzeichnet ist, dass man die Verbindung der Formel (II) aus dem Estergemisch der Formeln (I), (II), (III) trennt, oder in einem Zweistufenprozess Säure bzw. Säureanhydrid mit den Alkoholen der Formeln (IV) und (V) oder dessen oder Syntheseäquivalenten nacheinander zur Reaktion bringt, verbunden mit einem möglichen Reinigungsschritt nach Stufe 1 oder 2.

Falls die zur Herstellung der erfindungsgemäßen Bernsteinsäurealkylester Mischungen eingesetzte Bernsteinsäure aus bio-basierten Rohstoffen hergestellt wurde, z.B. durch einen mikrobiologischen Fermentationsprozess, so können Verunreinigungen in der Bernsteinsäure enthalten sein, die sich auch in den erfindungsgemäßen Mischungen wiederfinden können. Typische Verunreinigungen, die durch die eingesetzten Mikroorganismen in die Endprodukte gelangen können sind Stickstoff- und Schwefel-haltige Verbindungen.

Bevorzugt enthalten die erfindungsgemäßen Estermischungen weniger als 1000 ppm Massenanteil an Stickstoffatomen und weniger als 50 ppm Massenanteil an Schwefelatomen jeweils bezogen auf die Mischung. Besonders bevorzgt sind erfindungsgemäße Mischungen die 0,01 bis 750 ppm Massenanteil an Stickstoffatomen und 0,0001 bis 40 ppm Massenanteil an Schwefelatomen jeweils bezogen auf die Mischung enthalten.

Ebenfalls Gegenstand der vorliegenden Erfindung sind Estermischung enthaltend wenigstens zwei Bernsteinsäurealkylester der Formeln (I), (II) und (III) das dadurch gekennzeichnet ist, dass die Bernsteinsäurealkylester der Formeln (I), (II) und (III) von Biomasseressourcen stammen und in der Mischung 0,01 ppm bis 1000 ppm Massenanteil an Stickstoffatomen und 0,01 ppm bis 50 ppm Massenanteil an Schwefelatomen jeweils bezogen auf die Mischung enthalten ist.

Diese erfindungsgemäßen Estermischungen können hergestellt werden, indem man bei der Durchführung des erfindungsgemäßen Herstellungsverfahrens Alkohole der Formeln (IV) und (V) und Bernsteinsäure einsetzt die aus Biomasseressourcen stammen und wobei der Massenanteil an Stickstoffatomen bezogen auf die Gesamtmasse an eingesetzten Alkoholen und Bernsteinsäure im Bereich von 0,01 ppm bis 1000 ppm liegt und der Masseanteil an Schwefelatomen bezogen auf die Gesamtmasse an eingesetzten Alkoholen und Bernsteinsäure im Bereich von 0,01 ppm bis 50 ppm liegt.

Die Eignung der erfindungsgemäßen Bernsteinsäurealkylester Mischungen als Weichmacher wird durch das Vorhandensein der genannten geringen Mengen an typischen Verunreinigungen nicht negativ beeinflusst.

Die neuen Bernsteinsäureester Mischungen eignen sich hervorragend als Weichmacher für Kunststoffe.

Weiterer Gegenstand der Erfindung ist die Verwendung einer erfindungsgemäßen Bernsteinsäureester Mischung als Weichmacher für Kunststoffe.

Als Kunststoffe eignen sich z.B Polyvinylchlorid (PVC), Vinylchlorid-basierte Copolymere, Polyvinylidenchlorid, Polyvinylacetale, Polyvinylbutyral, Polyacrylate, Polymethacrylate, Polyalkylmethacrylate wie z.B. Poly(methylmethacrylat), Polyamide, Polyurethane, Polylactide, Polymilchsäuren, Polyvinylacetat, Cellulose und seine Derivate, Ethylenvinylacetate, Kautschukpolymere wie Acrylnitril-Butadien-Kautschuk, hydrierter Acrylnitril-Butadien-Kautschuk, Styrol-Butadien-Kautschuk, Chloroprenkautschuk, Ethylen-Propylen-Kautschuk, Acrylatkautschuk und Naturkautschuk. Bevorzugt werden die erfindungsgemäßen Bernsteinsäureester Mischungen als Weichmacher und Verarbeitungshilfen für PVC und Polyacrylate eingesetzt..

Die mit den neuen Weichmachern hergestellten Kunststoffe zeichnen sich durch bessere Dauergebrauchseigenschaften und eine längere Nutzungsdauer im Vergleich zu den aus dem Stand der Technik bekannten mit Weichmachern auf Basis von einkomponentigen Bernsteinsäureestern hergestellten Kunststoffen aus.

Gegenstand der Erfindung ist auch die Verwendung der erfindungsgemäßen Bernsteinsäureester Mischungen als Verarbeitungshilfen und Weichmacher in Klebstoffen, Klebstoffkomponenten, Klebdichtstoffen, Klebdichtstoffkomponenten, Dichtungsmassen, Dichtungsmassenkomponenten, oder Beschichtungsmassen, in Farben, Tinten oder Lacken, in Plastisolen inklusive PVC-Plastisolen, und als Weichmacher in Kunststoffen oder Kunststoffkomponenten, bevorzugt in Polyvinylchlorid.

Weiterer Gegenstand der vorliegenden Erfindung sind Weichmacherzubereitungen enthaltend eine erfindungsgemäße Bernsteinsäureester Mischung sowie gegebenenfalls weitere übliche Zusatzstoffe. Als solche Zusatzstoffe kommen beispielsweise weitere Weichmacher, Stabilisatoren, Antioxidantien, Gleitmittel, Füllstoffe, Pigmente, Flammschutzmittel, Lichtstabilisatoren, Treibmittel, Kicker, polymere Verarbeitungshilfsmittel, Schlagzähverbesserer, optische Aufheller, Antistatika oder Biostabilisatoren in Frage.

Als PVC-Typen kommen Suspensions-, Masse-, Mikrosuspensions- oder Emulsions-PVC in Frage. Dabei können die Gemische alleine oder in Kombination mit anderen Weichmachern eingesetzt werden. Die erfindungsgemäßen Weichmacher werden im allgemeinen zu 10 bis 200 Teilen, bevorzugt zu 20 bis 150 Teilen, jeweils auf 100 Teile Kunststoff eingesetzt.

Die neuen Weichmacherzubereitungen ermöglichen die Herstellung von Endprodukten mit guten Tieftemperatureigenschaften. Bevorzugt werden die erfindungsgemäßen Gemische zur Herstellung von Plastisolen, bevorzugt von Plastisolen auf Basis von PVC, eingesetzt. Eingesetzt in PVC Plastisolen erlauben die niedrigviskosen Bernsteinsäureestermischungen die Herstellung von niedrigviskosen, lagerstabilen Plastisolen.

Die unter Verwendung der erfindungsgemäßen Weichmacherzubereitungen hergestellten Kunststoffe, insbesondere das Polyvinylchlorid, können neben den erfindungsgemäßen Weichmacherzubereitungen auch noch andere, geeignete Hilfs- und Zusatzstoffe enthalten. Beispiele hierfür sind weitere Weichmacher, Stabilisatoren, Antioxidantien, Gleitmittel, Füllstoffe, Pigmente, Flammschutzmittel, Lichtstabilisatoren, Treibmittel, Kicker, polymere Verarbeitungshilfsmittel, Schlagzähverbesserer, optische Aufheller, Antistatika oder Biostabilisatoren.

Die vorliegende Erfindung betrifft weiterhin ein Verfahren zu Herstellung von weichgemachten Kunststoffen, insbesondere von weichgemachtem PVC, das dadurch gekennzeichnet ist, dass man in einem ersten Schritt PVC, insbesondere Emulsions- und Microsuspensions-PVC, bei 10 bis 60 °C mit der erfindungsgemäßen Weichmacherzubereitung und ggf. weiteren Hilf- und Zusatzstoffen vermischt, wobei auf 100 Teile Kunststoff 10 bis 200 Teile des erfindungsgemäßen Weichmachers eingesetzt werden. In einem zweiten Schritte wird dieses Plastisol in Form gebracht und bei Temperaturen von 140 bis 200°C zum Endartikel verarbeitet.

### Herstellungsbeispiele und technische Prüfung

Die folgenden Beispiele dienen der Erläuterung der vorliegenden Erfindung und sollen in keiner Weise als Einschränkung verstanden werden.

### 1. Herstellung einer erfindungsgemäßen Bernsteinsäureester Mischung

Synthese der Bernsteinsäureestergemische (Succinate) durch Veresterung von Bernsteinsäure mit zwei monofunktionellen Alkylalkoholen:
In einem mit Stickstoffgas inertisiertem 1 1 Mehrhalsrundkolben mit Wasserabscheider und Rückflusskühler, Innenthermometer und Rührer wurden 151 g Bernsteinsäure, 230 g 1-Oktanol, 283 g 1-Dekanol vorgelegt. Der Wasserabscheider wurde mit 1-Oktanol gefüllt. Nach Zugabe von 0,3 g Tetrabutyltitanat wurde der Ansatz unter Rühren zum Rückfluß erwärmt und gebildetes Reaktionswasser ausgekreist. Der Reaktionsverlauf wurde mittels Titration [Säurezahl] und Wasserabscheidung verfolgt. Nach Erreichen einer Säurezahl von kleiner gleich 1 wurde die Reaktion beendet. Nach Umbau der Apparatur wurde der Überschuß an Alkohol im Vakuum beginnend bei 20 mbar abdestilliert; die Sumpftemperatur betrug zum Ende der Destillation 185°C. Nach Abkühlen wurde der Katalysator mit Wasser und wässriger Natriumcarbonatlösung gewaschen. Anschließend wurden, beginnend bei 120°C und 20 mbar, die flüchtigen Bestandteile aus der organischen Phase destillativ entfernt. und die Mischung anwendungstechnisch als Weichmacher geprüft. Die Ausbeute an der Estermischung betrug 456 g (ca. 96 %, bezogen auf n-Octyl-n-decylsuccinat). Die Zusammensetzung nach GC Flächenprozenten war: 22,8% Di-n-octylsuccinat, 50,4% n-Octyl-n-decylsuccinat, 26,4% Di-n-decylsuccinat.

Die Vergleichsprodukte Di-n-octylsuccinat und Di-n-decylsuccinat wurden auf dem gleichen Wege hergestellt.

### Bestimmung der Härte:

Zur Bestimmung der Härte wurde eine weichmacherhaltige PVC-Pulvermischung auf einem Zweiwalzenstuhl homogenisiert und geliert; anschließend wurde der Compound zu Prüfplatten gepresst und die Härte mit einem Zwick Shorehärtegerät bestimmt. Die Härte (Shore A) wurde an glatten und ebenen Prüfkörpern der Dimension 6 mm x 40 mm x 50 mm gemessen. Niedrige Shore A Härten bedeuten weichere Produkte und sind ein Maß für die Effizienz der Weichmacher.

In einer Porzellanschale wurden 100 g Polyvinylchlorid (Vinnolit^{®} S4170, Vinnolit GmbH & Co. KG, Deutschland), mit 60 phr (parts per hundred resin) Weichmacher bzw. Weichmacherzubereitung und 3 phr PVC-Stabilisator (Ca/Zn-Carboxylat) mit einem Stab so gemischt, dass die flüssigen Bestandteile vom Pulver gut aufgenommen wurden und nicht an dem Gefäß haften. Die so erhaltene Pulvermischung wurde portionsweise in den Walzenspalt (0,7 mm) eines Zweiwalzenstuhles bei 165°C Walzentemperatur gegeben und homogenisiert und geliert. Nach der Fellbildung wurde der Walzenspalt auf 1 mm erweitert. Das Mischergebnis wurde durch häufiges Einschlagen des Walzfelles verbessert. Nach einer Misch- und Verarbeitungszeit von 10 Minuten wurde das Walzfell abgenommen. Nach Portionierung wurden Prüfkörper der Dimension 6 mm x 40 mm X 50 mm gepresst. Die Temperatur der Presse betrug 170 °C; die Presszeit betrug insgesamt 10 Minuten, davon 7 Minuten Aufheizphase mit einem Druck < 10 bar und 3 Minuten Presszeit unter Hochdruck > 100 bar. Nach Abkühlen unter Druck in einer Kühlpresse auf maximal 30°C wurden die Prüfkörper entformt. Von den Prüfkörpern wurde nach frühestens 24 Stunden Lagerung bei 23 °C die Shore A Härte mit einem Zwick Typ H04.3150 nach DIN 53505 an fünf verschiedenen Stellen unter Notierung des Mittelwertes bestimmt.

### Anwendungstechnische Beispiele

In den Beispielen wird die Shore A Härte eines Prüfköpers mit einem Gehalt von 60 phr Weichmacher aufgeführt. Eine niedrige Härte ist eine Aussage über die gute weichmachende Eigenschaft des Weichmachers.

Die Härte nach Entformung wurde nach 7 Tage Lagerung bei 23°C bestimmt. Die Ausgangshärte wurde nach 1 Tag Lagerung bei 100°C hängend im Ofen inklusive 1 weiteren Tag Lagerung liegend bei 23°C bestimmt. Eine weitere Härtebestimmung der Prüfkörper fand nach 7 Tagen Lagerung hängend im Ofen bei 100°C inklusive 1 Tag Lagerung liegend bei 23°C statt.

**Tabelle 1: Härte von weichmacherhaltigen PVC Prüfkörpern vor, während und am Ende von Ofenlagerungen bei 100°C**

| Shore A Härte | Di-n-octylsuccinat | n-Octyl-n-decylsuccinat | Di-n-Decylsuccinat |
|---|---|---|---|
| Härte nach Entformung | 70 | 75 | 81 |
| 1 Tag 100°C | 67 | 70 | 76 |
| 7 Tage 100°C | 73 | 71 | 78 |

Die oben aufgeführten Beispiele zeigen, dass das erfindungsgemäße Estergemisch n-Octy-n-Decyl-succinat gegenüber den Vergleichsbeispielen Di-n-octylsuccinat und Di-n-decylsuccinat den Vorteile der besser weichmachenden Wirkung nach einer Lagerzeit von 7 Tagen bei 100°C aufweist. Daneben weist es gegenüber dem Di-n-octylsuccinat eine geringere Änderung der Härte während der Ofenlagerung auf. Gegenüber dem Di-n-decylderivat zeigt sich eine deutliche Verbesserungen in der weichmachenden Wirkung. Aus Tabelle 1 lässt sich eine längere, effektivere Nutzungsdauer der Endprodukte bei Einsatz des Estergemisches ableiten.

## Patentansprüche

1. Mischung von Bernsteinsäurealkylestern **dadurch gekennzeichnet, dass** sie mindestens zwei Verbindungen ausgewählt aus den Formeln
R¹-OC(O)-CH₂-CH₂-C(O)O-R¹ (I)
R¹-OC(O)-CH₂-CH₂-C(O)O-R² (II)
und
R²-OC(O)-CH₂-CH₂-C(O)O-R² (III)
enthält,
worin
R¹ und R² jeweils für einen geradkettigen oder verzweigten Alkylrest stehen,
mit der Maßgabe, dass R¹ nicht gleich R² ist.

2. Estermischung gemäß Anspruch 1, wobei in den Formeln (I), (II) und (III)
R¹ und R² jeweils für einen geradkettigen oder verzweigten Alkylrest mit 1 bis 12 Kohlenstoffatomen stehen.

3. Estermischung gemäß Ansprüchen 1 und 2, **dadurch gekennzeichnet, dass** in den Formeln (I), (II) und (III) R¹ für n-Octyl und R² für n-Decyl steht.

4. Estermischung gemäß wenigstens einem der Ansprüche 1 bis 3, **dadurch gekennzeichnet, dass** sie die Verbindungen der Formeln (I), (II) und (III) enthält.

5. Estermischung gemäß wenigstens einem der Ansprüche 1 bis 4, **dadurch gekennzeichnet, dass** die Menge an Verbindung der Formel (I) 10 bis 50 Gew.-%, bevorzugt 15 bis 35 Gew.-% und ganz besonders bevorzugt 20 bis 30 Gew.-%, die Menge an Verbindung der Formel (II) 25 bis 75 Gew.-%, bevorzugt 35 bis 65 Gew.-% und ganz besonders bevorzugt 40 bis 60 Gew.-%, und die Menge an Verbindung der Formel (III) 10 bis 50 Gew.-%, bevorzugt 15 bis 35 Gew.-% und ganz besonders bevorzugt 20 bis 30 Gew.-%, jeweils bezogen auf 100 Prozent der Mischung, beträgt.

6. Estermischung gemäß wenigstens einem der Ansprüche 1 bis 3, **dadurch gekennzeichnet, dass** sie nur die Verbindungen (I) und (III) enthält.

7. Estermischung gemäß Anspruch 6, **dadurch gekennzeichnet, dass** die Menge an Verbindung der Formel (I) 15 bis 95 Gew.-% bevorzugt 25 bis 75 Gew.-% und ganz besonders bevorzugt 40 bis 60 Gew.-%, und die Menge an Verbindung der Formel (III) 85 bis 5 Gew.-%, bevorzugt 75 bis 25 Gew.-% und ganz besonders bevorzugt 60 bis 40 Gew.-%, jeweils bezogen auf 100 Prozent der Mischung, beträgt.

8. Bernsteinsäurealkylester der Formel
R¹-OC(O)-CH₂-CH₂-C(O)O-R² (II)
worin
R¹ und R² jeweils für einen geradkettigen oder verzweigten Alkylrest stehen,
mit der Maßgabe, dass R¹ nicht gleich R² ist.

9. Estermischung gemäß wenigstens einem der Ansprüche 1 bis 7, **dadurch gekennzeichnet, dass** die Bernsteinsäurealkylester der Formeln (I), (II) und (III) von Biomasseressourcen stammen und in der Mischung 0,01 ppm bis 1000 ppm Massenanteil an Stickstoffatomen und 0,01 ppm bis 50 ppm Massenanteil an Schwefelatomen jeweils bezogen auf die Mischung enthalten ist.

10. Verfahren zur Herstellung einer Estermischung gemäß Anspruch 4, **dadurch gekennzeichnet ist, dass** man in einem Verfahrensschritt oder in zwei aufeinander folgenden Verfahrensschritten zwei unterschiedliche monofunktionelle Alkohole der Formeln R¹-OH (IV) und R²-OH (V), worin R¹ und R² die in Anspruch 1 angegebene Bedeutung haben, bei einer Temperatur von 50 bis 250°C und gegebenenfalls bei einem Druck von 2 mbar bis 4 bar und gegebenenfalls in Gegenwart eines Katalysators mit Bernsteinsäure umsetzt und das gebildete Reaktionswasser durch geeignete Maßnahmen, beispielsweise Destillation, aus der Mischung entfernt.

11. Verwendung einer Estermischung oder eines Bernsteinsäurealkylesters gemäß wenigstens einem der Ansprüche 1 bis 9 als Weichmacher für Kunststoffe und Kunststoffkomponenten.

12. Verwendung einer Estermischung oder eines Bernsteinsäureesters gemäß wenigstens einem der Ansprüche 1 bis 9 als Verarbeitungshilfsmittel und/oder Weichmacher in Klebstoffen, Klebstoffkomponenten, Klebdichtstoffen, Klebdichtstoffkomponenten, Dichtungsmassen, Dichtungsmassenkomponenten, oder Beschichtungsmassen, in Farben, Tinten oder Lacken, sowie in Plastisolen.

13. Weichmacherzubereitung enthaltend eine Estermischung oder einen Bernsteinsäurealkylester gemäß wenigstens einem der Ansprüche 1 bis 9.

14. Verfahren zu Herstellung von weichgemachten Kunststoffen, **dadurch gekennzeichnet, dass** man in einem ersten Schritt PVC bei 10 bis 50 °C mit einer Weichmacherzubereitung gemäß Anspruch 13 und gegebenenfalls weiteren Hilf- und Zusatzstoffen vermischt, wobei auf 100 Teile Kunststoff 10 bis 200 Teile der Weichmacherzubereitung eingesetzt werden und in einem zweiten Schritte das so hergestellte Plastisol in Form gebracht und bei Temperaturen von 140 bis 200°C zum Endartikel verarbeitet wird.

15. Plastisol enthaltend mindestens einen Kunststoff und eine Weichmacherzubereitung gemäß Anspruch 13.
